# EUROPÄISCHE PATENTSCHRIFT

(11) **EP 1 459 062 B1**
(45) Veröffentlichungstag und Bekanntmachung des Hinweises auf die Patenterteilung: **24.05.2006**
(21) Anmeldenummer: 02792723.5
(22) Anmeldetag: 17.10.2002
(51) Int. Cl.: G01N 33/00, G08B 17/117, A62C 3/02, G01N 27/416

(54) **VERFAHREN UND VORRICHTUNG ZUR MESSUNG DES SAUERSTOFFGEHALTES IN EINEM ABGESCHLOSSENEN ZIELRAUM**
METHOD AND DEVICE FOR MEASURING OXYGEN CONTENT IN A CLOSED TARGET SPACE
PROCEDE ET DISPOSITIF DE MESURE DE LA TENEUR EN OXYGENE DANS UN ESPACE CIBLE FERME

(30) Priorität: 28.12.2001 DE 10164293
(43) Veröffentlichungstag der Anmeldung: 22.09.2004
(73) Patentinhaber: Wagner Alarm- und Sicherungssysteme GmbH, 30853 Langenhagen (DE)
(72) Erfinder: WAGNER, Ernst, Werner, 29308 Winsen/Aller (DE)
(74) Vertreter: Rupprecht, Kay
(86) Internationale Anmeldenummer: PCT/EP2002/011648
(87) Internationale Veröffentlichungsnummer: WO 2003/056325

(56) Entgegenhaltungen:
- EP-A- 1 030 279
- EP-A- 1 092 975
- DE-A- 10 018 991
- DE-A- 19 624 976
- DE-A- 19 811 851
- US-A- 3 984 826
- US-A- 4 177 787
- US-A- 5 552 775
- US-A- 5 844 148

## Beschreibung

Die vorliegende Erfindung betrifft ein Verfahren zum Messen des Sauerstoffgehaltes in einem abgeschlossenen Zielraum, insbesondere zur Überwachung von Intertisierungsniveaus bei einer Inertgasvorrichtung zur Brandvermeidung und/oder -löschung, sowie eine Vorrichtung zur Durchführung des Verfahrens.

Das Dokument DE 198 11 851 offenbart ein Inertisierungsverfahren zur Minderung des Risikos und zum Löschen von Bränden in geschlossenen Räumen sowie eine Vorrichtung zur Durchführung des Verfahrens. Hierbei ist ein zweistufiges Inertisierungsverfahren vorgesehen, bei dem in dem geschlossenen Raum zunächst der Sauerstoffgehalt auf ein bestimmtes Grundinertisierungsniveau und im Falle eines Brandes auf ein bestimmtes Vollinertisierungsniveau weiter abgesenkt wird. Zur Steuerung des Inertisierungsverfahrens ist ein Sauerstoffmessverfahren vorgesehen, bei dem über ein Rohrleitungssystem repräsentative Teilmengen der Raumluft aktiv angesaugt und einer Messkammer mit einem Sauerstoffsensor zugeführt werden. Ferner ist vorgesehen, dass in jeder Teilmenge der Raumluft Brandkenngrößen gemessen werden, und dass bei Bedarf und in Erwiderung auf die Messung der Brandkenngröße eine Vollinertisierung des Zielraums initiiert wird.

Das Dokument US-A-4,177,787 offenbart eine Vorrichtung sowie ein Verfahren zum Erfassen der Alterung eines Sauerstoffmelders in einem Abgasrohr einer Verbrennungsmaschine. Hierbei ist vorgesehen, neben einem Sauerstoffsensor zur Überwachung bzw. Erfassung des Treibstoff-Verbrennungsgrades in den Abgasen der Verbrennungsmaschine ferner einen Referenzsensor vorzusehen, der ebenfalls die Sauerstoffkonzentration in den Abgasen erfasst. Die beiden von den jeweiligen Sauerstoffsensoren unabhängig erfassten Werte werden anschließend miteinander verglichen. Dieser Vergleich liefert eine Aussage darüber, ob der Sauerstoffsensor einwandfrei arbeitet, oder ob dieser in Folge von Ablagerungen etc. bereits soweit gealtert ist, dass die von ihm abgegebenen Messwerte einen Fehler-Toleranzbereich überschreiten.

Das Dokument EP-A-1 092 975 offenbart ein Verfahren zum Messen von Sauerstoff in einer Auspuffleitung eines Verbrennungsmotors. Mit dem Ziel, eine Sensoranordnung sowie ein Verfahren bereitzustellen, durch welche bzw. durch welches mit niedrigem Aufwand zusätzliche Messdaten zur Optimierung eines in der Verbrennungskammer eines Verbrennungsmotors zu zündenden Luft-Treibstoffgemisches gewonnen werden können, ist hierzu vorgesehen, eine Sensorelektronik sowie einen Gassensor einzusetzen, der mindestens eine einen konstanten Sauerstoffpartialdruck repräsentierende Referenzelektrode, einen Sauerstoffionen-leitenden Festelektrolyten und mindestens zwei Messelektroden auf einer Messgasseite aufweist.

In geschlossenen Räumen, deren Einrichtungen sensibel auf Wassereinwirkung reagieren, wie etwa EDV-Bereiche, elektrische Schalt- und Verteilerräume oder Lagerbereiche mit hochwertigen Wirtschaftsgütern, werden in zunehmendem Maße zur Minderung des Risikos und zum Löschen von Bränden sogenannte Inertisierungsverfahren eingesetzt. Die bei diesen Verfahren resultierende Löschwirkung beruht auf dem Prinzip der Sauerstoffverdrängung. Normale Umgebungsluft setzt sich bekanntlich zu 21 Vol.-% aus Sauerstoff, 78 Vol.-% aus Stickstoff und 1 Vol.-% aus sonstigen Gasen zusammen. Zum Löschen und Vermeiden von Bränden wird durch Einleiten eines sauerstoffverdrängenden Inertgases, wie etwa reiner Stickstoff, die Inertgaskonzentration in dem betreffenden Raum erhöht und der Sauerstoffanteil verringert. Viele Stoffe brennen nicht mehr, wenn der Sauerstoffanteil unter 15 - 18 Vol.-% absinkt. Abhängig von den in dem betreffenden Raum vorhandenen brennbaren Materialien kann ein weiteres Absinken des Sauerstoffanteils auf beispielsweise 12 Vol.-% erforderlich sein.

Aus der Patentschrift DE 198 11 851 C2 ist ein Inertisierungs-verfahren bekannt, welches ein effektives Löschen eines Brandes bei möglichst geringer Lagerkapazität für die Inertgasflaschen ermöglicht. Bei diesem Verfahren wird der Sauerstoffgehalt in dem geschlossenen Zielraum auf ein Grundinertisierungsniveau von beispielsweise 16 VoL-% reduziert, in einem Brandfall erfolgt dann eine sehr schnelle Vollinertisierung auf beispielsweise 12 Vol.-% oder darunter.

Eine Inertgasvorrichtung zur Brandvermeidung und/oder -löschung zur Durchführung des genannten Inertisierungsverfahrens weist zunächst folgende Bauteile auf: Eine Sauerstoffmessvorrichtung zum Messen des Sauerstoffgehaltes in dem zu überwachenden Zielraum; eine Branderkennungsvorrichtung zum Detektieren einer Brandkenngröße in der Raumluft des Zielraumes; eine Steuerung zur Auswertung der Daten der Sauerstoffmessvorrichtung und des Brandkenngrößendetektors und zur Ablaufsteuerung des Inertisierungsverfahrens; und eine Anlage zur Produktion und zum plötzlichen Einleiten von Inertgas in den Zielraum.

Unter dem Begriff Brandkenngröße werden physikalische Größen verstanden, die in der Umgebung eines Entstehungsbrandes meßbaren Veränderungen unterliegen, z.B. die Umgebungstemperatur, der Feststoff- oder Flüssigkeits- oder Gasanteil in der Umgebungsluft (Bildung von Rauch in Form von Partikeln oder Aerosolen oder Dampf) oder die Umgebungsstrahlung.

Die Sauerstoffmessvorrichtung dient dazu, das Grundinertisierungsniveau im Zielraum einzustellen. Wenn ein Schwellwert der Sauerstoffkonzentration überschritten ist - beispielsweise aufgrund einer Leckage im Zielraum - gibt die Steuerung einen Befehl an eine spezielle Anlage zum Einleiten von Inertgas in den Raum, so daß der Sauerstoffanteil reduziert wird. Die Sauerstoffmessvorrichtung signalisiert, wenn der Schwellwert des Grundinertisierungsniveaus wieder erreicht ist. Die Lage des Grundinertisierungsniveaus ist dabei abhängig von Eigenschaften des Raumes.

Wird jedoch über den Detektor für Brandkenngrößen eine Brandkenngröße nachgewiesen, so erhält die Anlage den Befehl, den Raum mit Inertgas zu fluten, bis die Sauerstoffkonzentration im Zielraum auf ein bestimmtes Vollinertisierungsniveau reduziert ist.

Für ein sicheres Steuern des Verfahrens ist bei einer derartigen Inertgasvorrichtung zur Brandvermeidung und/oder löschung das Messen des Sauerstoffgehaltes im Zielraum ein wesentlicher Punkt. Im Stand der Technik werden zum Messen der Sauerstoffkonzentration im Zielraum punktförmige Sauerstoffsensoren eingesetzt, die Meßwerte des Sauerstoffgehalt in Form eines analogen Signals an die Steuerung abgeben. Sehr verbreitet sind 4 - 20 mA-Stromschnittstellen, wobei 4 mA eine Konzentration von 0 Vol.-% Sauerstoff und 20 mA das Ende des Messbereiches (z.B. 25 Vol.-% Sauerstoff) entsprechen. Der Einsatz punktförmiger Sauerstoffsensoren hat den Nachteil, daß eine große Anzahl derartiger Sensoren im Zielraum notwendig sind, um eine repräsentative Aussage über den Sauerstoffgehalt der Raumluft zu erhalten. Dies erfordert eine entsprechend aufwendige Verkabelung zwischen den einzelnen im Zielraum verteilten Sensoren und der eigentlichen Steuerung.

Des weiteren braucht die Steuerung eine entsprechend hohe Anzahl analoger Schnittstellen. Dies zieht insbesondere einen hohen und teuren Hardwareaufwand nach sich.

Als besonders nachteilig erweist sich aber auch, daß die Steuerung kontinuierlich eine große Anzahl von Signalen verarbeiten muß. Insbesondere ein Bilden von Mittelwerten, ein Abschätzen von Fehlem und der Vergleich mit voreingestellten Schwellenwerten sind hier notwendige Routinen, die zur Steuerung des Inertisierungsverfahrens unbedingt notwendig sind. Nur anhand der verarbeiteten Daten der Sauerstoffsensoren kann die Anlage zum Einleiten von Inertgas, eine Frischluftzufuhr oder ein Lüfter für die Luftumwälzung im Zielraum angesteuert werden. Dadurch wird die Signalverarbeitung in der Steuerung sehr aufwendig, was eine hohe Komplexität des Software nach sich zieht.

Auf der Grundlage der geschilderten Problemstellung liegt der vorliegenden Erfindung die Aufgabe zugrunde, ein Verfahren zum Messen des Sauerstoffgehaltes in einem Zielraum anzugeben, welches ein effektives, sicheres und repräsentatives Bestimmen der Sauerstoffkonzentration unter möglichst geringem Aufwand an die Instrumentierung und Signalverarbeitung ermöglicht.

Diese Aufgabe wird bei einem Verfahren zum Messen des Sauerstoffgehaltes der eingangs genannten Art mit folgenden Verfahrensschritten gelöst: Zunächst wird über eine Reihe von Ansaugöffnungen eines Ansaugrohrsystems eine Luftprobe aus dem Zielraum entnommen, um anschließend mit einem Sauerstoffmelder die Sauerstoffkonzentration der angesaugten Luftprobe zu bestimmen.

Die erfindungsgemäße Lösung weist eine ganze Reihe wesentlicher Vorteile gegenüber der aus der Inertgaslöschtechnik bekannten und vorstehend erläuterten Verfahren zum Messen des Sauerstoffgehaltes in einem Zielraum auf. Durch ein Ansaugen über Öffnungen des Ansaugrohrsystems werden Luftproben aus verschiedenen Ansaugöffnungen vermischt. Dadurch entspricht die Sauerstoffkonzentration der angesaugten Luftprobe automatisch einem Mittelwert der Sauerstoffkonzentration des Zielraumes, somit entfällt die aufwendige Mittelwertsbildung bei der Signalverarbeitung in einer Steuerung. Eine Software zur Aus- bzw. Bewertung der Meßwerte fällt in einer entsprechend einfacheren Ausführung aus. Ferner ist bei dem erfindungsgemäßen Verfahren das Überwachungs- bzw. Meßvolumen wesentlich größer, als bei punktförmig angeordneten Sauerstoffsensoren der bekannten Art. Dies bringt insbesondere Kostenvorteile bei der Anschaffung, der Installation und der Wartung der Vorrichtung zum Messen des Sauerstoffgehaltes in dem Zielraum und letztendlich der gesamten Inertgasvorrichtung zur Brandvermeidung und/oder -löschung.

Die vorstehend genannte Aufgabe wird ferner durch eine Vorrichtung zur Durchführung dieses Verfahrens gelöst, die zunächst wenigstens ein Ansaugrohrsystem zum Ansaugen einer Luftprobe über verschiedene Ansaugöffnungen aus dem zu überwachenden Zielraum aufweist.

Die efindungsgemäBe Vorrichtung verwirklicht in idealer Weise die Verbindung des erfindungsgemäßen Verfahrens mit einer Sauerstoffmessvorrichtung. Von Vorteil ist insbesondere, daß hierbei auf den Einsatz einer Vielzahl von punktförmigen Sauerstoffsensoren im Zielraum verzichtet werden kann. Stattdessen ist wenigstens ein Ansaugrohrsystem zum Ansaugen einer Luftprobe über verschiedene Ansaugöffnungen aus dem zu überwachenden Zielraum vorgesehen. Somit entfällt auch ein aufwendiges Verkabeln der bisherigen punktförmigen Sauerstoffsensoren mit der Steuerung. Da bei der Steuerung nur noch eine analoge Schnittstelle für die Sauerstoffmessvorrichtung zur Verfügung gestellt werden muß, kann diese mit geringem Hardwareaufgebot realisiert werden. Des weiteren ist die Signalverarbeitung in der Steuerung deutlich vereinfacht, da nicht mehr eine hohe Anzahl von Signalen einzelner Sauerstoff-Sensoren verarbeitet werden muß. Demzufolge kann auch die Software zur Signalverarbeitung entsprechend einfach ausgeführt sein. Durch die erfindungsgemäße Lösung ist es somit möglich, den Sauerstoffgehalt mit geringem Aufwand bezüglich der Instrumentierung und Signalverarbeitung zu messen, was insbesondere Kostenvorteile bei der Anschaffung und Wartung der gesamten Inertgasvorrichtung bringt.

Vorteilhafte Weiterbildungen des Verfahrens sind in den Unteransprüchen 2 bis 7 angegeben, und der Vorrichtung in den Ansprüchen 9 bis 12.

Vorzugsweise enthält das Sauerstoffmessverfahren die folgende weiteren zwei Verfahrensschritte, welche nach dem zweiten Verfahrensschritt - der Bestimmung der Sauerstoffkonzentration der angesaugten Luftprobe mit dem Sauerstoffmelder - durchgeführt werden: Nach dieser Weiterbildung wird zunächst der Meßwert der Sauerstoffkonzentration der angesaugten Luftprobe im Sauerstoffmelder mit eingestellten Schwellenwerten verglichen und anschließend erfolgt beim Überschreiten des eingestellten Schwellenwertes durch ein Einleiten von Inertgas in den Zielraum eine Absenkung der Sauerstoffkonzentration. Somit wird durch das kontinuierliche Messen des Sauerstoffgehaltes das Inertisierungsverfahren an mögliche Leckagen des Zielraumes angepaßt. Der Vorteil dieser Weiterbildung liegt insbesondere darin, daß bei der erfindungsgemäßen Sauerstoffmessverfahren eine gewisse eigene "Intelligenz" vorliegt, weil das Verfahren einen Vergleich mit vorbestimmten Schwellenwerten von selbst vornimmt. Erst bei Überschreiten eines Schwellenwertes wird dies der Steuerung in der Zentrale gemeldet. Somit wird nicht nur der Datenverkehr zwischen der Vorrichtung zur Durchführung des erfindungsgemäßen Sauerstoffmessverfahrens und der Steuerung der Inertgasvorrichtung zur Brandvermeidung und/oder -löschung sondern auch die Signalverarbeitung in der Steuerung deutlich reduziert. Durch diese sogenannte "verteilte Intelligenz" kann eine Signalverarbeitung zwischen der Steuerung und dem angeschlossenen Sauerstoffmelder aufgeteilt werden. Dies ermöglicht es, den Softwareaufwand und insbesondere den Anschaffungspreis sowie den Wartungsaufwand der Steuerung einer Inertgasvorrichtung zur Brandvermeidung und/oder -löschung deutlich zu reduzieren.

Vorzugsweise wird ein Detektor für Brandkenngrößen in das erfindungsgemäße Verfahren zum Messen des Sauerstoffgehaltes in dem Zielraum integriert. Dieser Detektor gibt im Brandfall ein Signal für die Vollinertisierung ab. Diese Weiterbildung ist die verfahrenstechnische Umsetzung der Verbindung einer bekannten aspirativen Branderkennungsvorrichtung mit der Inertgaslöschtechnik. Hierbei wird unter einer aspirativen Branderkennungsvorrichtung eine Branderkennungsvorrichtung verstanden, die über ein Ansaugrohrsystem, Rohrleitungssystem oder Kanalsystem an einer Vielzahl von Stellen eine repräsentative Teilmenge der Raumluft aktiv ansaugt und diese Teilmengen dann einem Detektor zum Erfassen einer Brandkenngröße zuleitet. Durch die Integration des Detektors für Brandkenngrößen in der Vorrichtung zur Durchführung des erfindungsgemäßen Verfahrens wird hier neben der Sauerstoffmessvorrichtung eine aspirative Branderkennungsvorrichtung eingerichtet. Von Vorteil ist, daß zur Umsetzung einer derartigen aspirativen Branderkennungsvorrichtung auf bereits vorhandene Komponenten zurückgegriffen werden kann. Dies ermöglicht es, den Zielraum ohne besonderen Aufwand mit einer aspirativen Branderkennungsvorrichtung auszurüsten und die Branderkennung zu verbessern.

In einer besonders vorteilhaften Ausführungsform der erfindungsgemäßen Lösung ist vorgesehen, daß die im Detektor nachgewiesenen Brandkenngrößen Rauch in Form von Partikeln, Aerosolen oder Dampf und wenigstens ein Brandgas sind. Dadurch wird erreicht, daß die Branderkennungsvorrichtung, welche mit der erfindungsgemäßen Sauerstoffmessvorrichtung ausgestattet ist, bezüglich den Kenngrößen, die bekannterweise typisch für einen Brand sind, besonders sensibel reagiert. Damit kann ein Brand vorzugsweise schon bereits bei der Entstehung erkannt und die Inertgasvorrichtung zur Brandvermeidung und/oder -löschung alarmiert werden.

Eine mögliche Realisierung der erfindungsgemäßen Sauerstofmiessvorrichtung besteht darin, daß das im Detektor nachgewiesene Brandgas CO oder CO₂ ist. Der Vorteil dieser Ausführungsform besteht insbesondere darin, daß so die Branderkennungsvorrichtung auf Brandkenngrößen besonders sensibilisiert ist und dadurch auch in der Lage ist, beispielsweise zwischen einem tatsächlichen Brand und Zigarettenrauch oder ähnlichen, rauchartigen aber nicht brandkennzeichnenden Größen zu unterscheiden. Selbstverständlich sind hier aber auch andere Ausführungsformen denkbar.

In einer vorteilhaften Ausführungsform wird das Messen der Luftqualität über einen CO- bzw. CO₂-Sensor in das Verfahren integriert, wobei die Frischluftzufuhr des Zielraumes in Abhängigkeit von dem Signal des CO- bzw. CO₂-Sensors gesteuert wird. Auch hier kommen die bereits oben im Zusammenhang mit dem Sauerstoffmelder diskutierten Vorteile zum Tragen. Insbesondere entfällt bei dieser vorteilhaften Weiterbildung der Einsatz einer Vielzahl im Zielraum verteilter, punktförmig messender CO- bzw. CO₂-Sonsoren und somit auch ein entsprechend hoher Hard- und Softwareaufwand in der die Signale verarbeitenden Steuerung.

In der Erfindung ist ein Verfahrensschritt integriert, bei dem mittels eines Referenzsauerstoffmelders in der angesaugten Luftprobe die Sauerstoffkonzentration bestimmt wird. Dies erfolgt insbesondere unabhängig von der Messung des zuvor genannten Sauerstoffmelders. Der Referenzsauerstoffmefder ist dabei in dem Luftstrom der angesaugten Luftprobe dauerhaft angeordnet. Denkbar wäre hierbei beispielsweise, dass sich der Referenzsauerstoffmelder in unmittelbarer Nähe des Sauerstoffmelders befindet. Der mittels des Referenzsauerstoffmetders erfasste Messwert der Sauerstoffkonzentration wird anschließend mit dem Messwert der Sauerstoffkonzentration verglichen, weicher zur selben Zeit mit dem Sauerstoffsensor in dem Luftstrom aufgenommen wurde. Dabei ist vorgesehen, dass ein Störungssignal abgegeben wird, wenn es sich beim Vergleich der beiden Messwerte zeigt, dass die Abweichung des mittels des Sauerstoffmelders ermittelten Wertes der Sauerstoffkonzentration von dem mittels des Referenzsauerstoffmelders ermittelten Wert der Sauerstoffkonzentration über einen zuvor festgelegten Toleranzwert abweicht Hierbei ist denkbar, dass der Vergleich der Messwerte bzw. die Abgabe des Störungssignals in bzw. von dem Sauerstoffmelder oder in bzw. von dem Referenzsauerstoffmelder erfolgt. Selbstverständlich sind hier aber auch andere Lösungen denkbar.

In der Erfindung ist der Referenzsauerstoffmeider - im Gegensatz zum Sauerstoffmelder - in der Regel normalerweise ausgeschaltet ist. Der Referenzsauerstoffmelder wird dabei nur in regelmäßigen zeitlichen Abständen, wie beispielsweise einmal am Tag oder einmal pro Woche, eingeschaltet. Nach dem Einschalten wird eine Mindestheizzeit abgewartet, bis die Sauerstoffkonzentration in dem Luftstrom bestimmt werden kann. Der Vorgang des Einschaltens könnte dabei über ein Signal erfolgen, welches etwa von einer Zeitschaltuhr erzeugt wird. Denkbar wäre hingegen auch, dass das Einschalten auf Tastendruck, beispielsweise bei Wartungen, geschieht Dadurch, dass der Referenzsauerstoffmelder nur kurzzeitig eingeschaltet ist, kann in besonders einfacher und leicht zu realisierender Weise eine frühzeitige Alterung des Referenzsauerstoffmelders verhindert werden.

Besonders bevorzugt verwendet man das Störungssignal, weiches abgegeben wird, wenn es sich beim Vergleich der beiden Messwerte zeigt, dass die Abweichung des mittels des Sauerstoffmelders ermittelten Wertes der Sauerstoffkonzentration von dem mittels des Referenzsauerstoffmelders ermittelten Wert der Sauerstoffkonzentration über einen zuvor festgelegten Toleranzwert abweicht, um damit zu bewirken, dass der Referenzsauerstoffmelder dauerhaft eingeschaltet bleibt und somit kontinuierlich Messwerte der Sauerstoffkonzentration der angesaugten Luftprobe liefert. Diese Messwerte werden dann anstelle der des Sauerstoffmelders ausgewertet. Damit kann erreicht werden, dass Messunsicherheiten, deren Ursache insbesondere in der Alterung des in der Regel kontinuierlich betriebenen Sauerstoffmelders liegt, ausgeschlossen werden können.

Als vorteilhafte Weiterbildung zur erfindungsgemäßen Vorrichtung ist vorgesehen, dass eine Einstellung von Inertisierungsniveaus im Zielraum und eine Steuerung der Frischluftzufuhr bzw. eines Lüfters über eine Steuerung erfolgt, wobei ferner wenigstens ein Sauerstoffmelder zum Messen der Sauerstoffkonzentration in einer aus dem Zielraum entnommenen Luftprobe und
wenigstens ein Detektor zum Erkennen von Brandkenngrößen in einer von einem der Ansaugrohrsysteme aus dem Zielraum genommenen Luftprobe vorgesehen sind. Hierbei handelt es sich um eine besonders leicht zu realisierende Ausführungsform der efindungsgemä-Ben Vorrichtung, bei der in vorteilhafter Weise die bei der Inertgasvorrichtung zur Brandvermeidung und/oder -löschung eingesetzten messtechnischen Komponenten lediglich zur Analyse der aus dem Zielraum genommenen Luftprobe eingesetzt werden.

Besonders bevorzugt sind wenigstens ein CO- bzw. CO₂-Sensor zum Messen der Luftqualität in einer von einem der Ansaugrohrsysteme aus dem Zelraum genommenen Luftprobe angeordnet. Hierdurch kann die erfindungsgemäße Lösung derart weitergebildet werden, dass auch die Luftqualität mittels der erfindungsgemäßen Inertgasvorrichtung zur Brandvermeidung und/oder -löschung überwacht werden kann.

Vorzugsweise sind in einem Ansaugrohrsystem ein Sauerstoffmelder zusammen mit einem Detektor zum Erkennen von Brandkenngrößen und bzw. oder einem CO- bzw. CO₂-Sensor integriert. Dadurch kann in vorteilhafter Weise die Anzahl der bei der Inertgasvorrichtung zur Brandvermeidung und/oder -löschung eingesetzten Komponenten reduziert werden. Dieses bringt insbesondere weitere Kostenvorteile bei der Anschaffung, Installation und Wartung einer Inertgasvorrichtung zur Brandvermeidung und/oder -löschung.

Eine mögliche Realisierung besteht darin, daß als Sauerstoffmelder elektrochemische Zellen aus Zirkondioxid eingesetzt werden. Sauerstoffsensoren auf der Basis von Zirkondioxid sind insbesondere aus der Fahrzeugtechnik bekannt und werden dort in Katalysatoren zum Messen des Sauerstoffgehaltes in Abgasen eingesetzt. Die Sensoren gelten als zuverlässige, sensible, robuste und wartungsarme Bauteile. Durch den Einsatz von Standardkomponenten in der erfindungsgemäßen Vorrichtung kann die Umsetzung der Erfindung besonders kosteneffizient erfolgen.

In der erfindungsgemäßen Vorrichtung ist vorgesehen, dass neben dem Sauerstoffmelder noch ein Referenzsauerstoffmelder zum Messen der Sauerstoffkonzentration der aus dem Zielraum entnommenen Luftprobe eingesetzt wird. Dieser dient als Referenz zum Sauerstoffmelder und sitzt ständig im Luftstrom, jedoch ist der Melder normalerweise ausgeschaltet. Dadurch wird die Alterung des Referenzsauerstoffmelders vermieden. Der Melder wird in regelmäßigen Abständen (z.B. einmal am Tag oder einmal pro Woche) eingeschaltet. Das Signal zum Einschalten des Referenzmelders wird beispielsweise von einer Zeitschaltuhr erzeugt. Es kann auch auf Tastendruck, etwa bei Wartungen, erzeugt werden. Nach dem einschalten des Referenzsauerstoffmelders wird die Mindestheizzeit abgewartet. Danach werden beide Messwerte des Sauerstoffmelders und des Referenzmelders verglichen. Ist der Unterschied zwischen beiden Messwerten größer als eine vorgegebene Schwelle, wird eine Störung gemeldet, und der Referenzsauerstoffmelder wird nicht mehr ausgeschaltet. Seine Messwerte werden anstelle des gealterten Sauerstoffmelders ausgewertet.

Im folgenden wird ein bevorzugtes Ausführungsbeispiel der erfindungsgemäßen Vorrichtung zum Messen des Sauerstoffgehaltes in einem abgeschlossenen Zielraum zur Überwachung von Inertiesierungsniveaus bei einer Inertgasvorrichtung zur Brandvermeidung und/oder - löschung anhand von Zeichnungen näher erläutert.

Es zeigen:
- Fig. 1: ein schematisches Blockdiagramm eines Ausführungsbeispiels der erfindungsgemäßen Vorrichtung zum Messen des Sauerstoffgehaltes in einer Inertgasvorrichtung zur Brandvermeidung und/oder -löschung; und
- Fig. 2: eine schematische Darstellung der erfindungsgemäßen Vorrichtung zum Messen des Sauerstoffgehaltes in einem geschlossenen Zielraum.

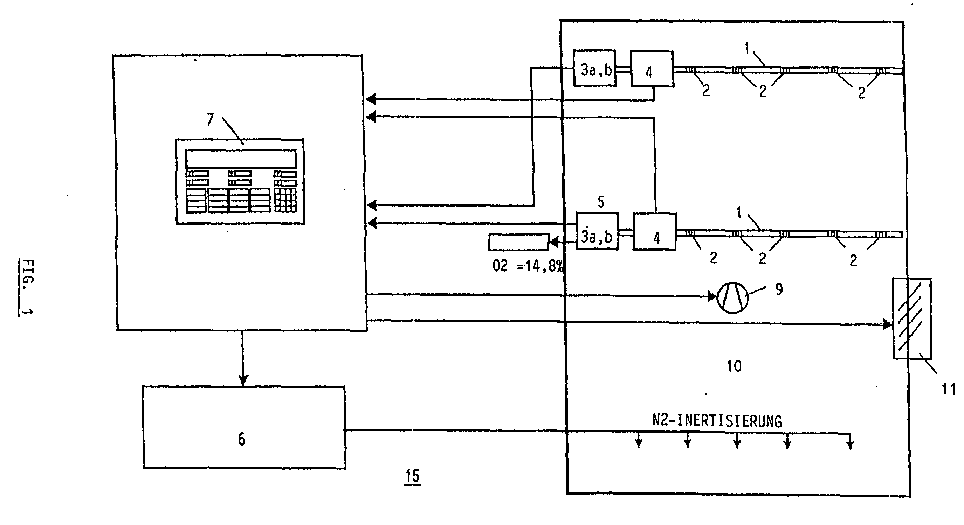

Fig. 1 zeigt ein schematisches Blockdiagramm eines Ausführungsbeispieles der erfindungsgemäßen Vorrichtung zum Messen des Sauerstoffgehaltes in einer Inertgasvorrichtung zur Brandvermeidung und/oder -löschung 6. Die Inertgasvorrichtung zur Brandvermeidung und/oder -löschung 6 dient zur Brandvermeidung und -löschung des geschlossenen Zielraumes 10. In dem Zielraum 10 sind zwei Ansaugrohrsysteme 1 zum Ansaugen von Luftproben über verschiedene Ansaugöffnung 2 angeordnet. Die Ansaugrohr-systeme 1 sind jeweils mit einem Ansaugmelder 8 ausgestattet, in dem die Luftproben aus dem Zielraum 10 einem Sauerstoffmelder 3 und einem Detektor 4 zur Erkennung von Brandkenngrößen bzw. einem CO- bzw. CO₂-Sensor 5 zugeführt werden. Bei der in Fig. 1 dargestellten Ausführungsform sind zwei Ansaugrohrsysteme 1 dargestellt, wobei eines der Rohrsysteme 1 unterhalb der Decke des Zielraumes 10, gegebenenfalls mit bis 1 m Abstand zur Decke, und das andere Rohrsystem 1 vorzugsweise in Einatmungshöhe, d.h. etwa 1,5 m über dem Boden, montiert ist.

Der Sauerstoffmelder 3a bestimmt die Sauerstoffkonzentration der jeweiligen Luftprobe und vergleicht den Meßwert mit eingestellten Schwellenwerten. Beim Überschreiten des eingestellten Schwellenwertes gibt der Sauerstoffmelder 3a über eine Datenleitung einer Steuerung 7 eine Meldung, welche ein Einleiten von Inertgas in den Zielraum 10 und ein Absenken der Sauerstoffkonzentration bewirkt. Die Steuerung 7 signalisiert hierzu eine Anlage zur Produktion und zum Einleiten von Inertgas 6 eine Inertisierung des Zielraumes 10 vorzunehmen.

Aufgrund der kontinuierlichen Entnahme von Luftproben aus dem Zielraum 10 durch die aspirative Ansaugvorrichtung wird kontinuierlich der Sauerstoffgehalt der Raumluft in dem Sauerstoffmelder 3a gemessen. Sobald der Meßwert der Sauerstoffkonzentration der kontinuierlich angesaugten Luftprobe im Sauerstoffmelder 3a mit einem eingestellten Schwellenwert übereinstimmt, erhält die Steuerung 7 eine entsprechende Meldung, die bewirkt, daß die weitere Inertisierung eingestellt wird.

In der in Fig. 1 dargestellten Ausführungsform wird im Detektor 4 zur Erkennung von Brandkenngrößen neben Rauch in Form von Partikeln, Aerosolen oder Dampf auch wenigstens ein Brandgas, wie etwa CO oder CO₂, nachgewiesen. Durch die Verwendung von wenigstens zwei unterschiedliche Brandkenngrößen, welche unabhängig voneinander einen Brand im Zielraum 10 nachweisen können, kann eine optimale Redundanz und ein entsprechende Ausfallsicherheit der Inertgasvorrichtung zur Brandvermeidung und/oder -löschung 6 verwirklicht werden. Somit wird insbesondere auch erreicht, daß der Detektor 4 in der Lage ist, zwischen einem tatsächlichen Brand und Zigarettenrauch oder ähnlichen, rauchartigen aber nicht brandkennzeichnenden Größen zu unterscheiden.

Bei einem weiteren Ansaugrohrsystem 1 gemäß Figur 1 sind in dem Ansaugmelder ein CO- bzw. CO₂-Sensor 5 und ein Detektor 4 zum Erkennen von Brandkenngrößen integriert. Der CO- bzw. CO₂-Sensor 5 überwacht die Luftqualität des Zielraumes 10, indem er den CO- bzw. CO₂-Gehalt der über das Ansaugrohrsystem entnommenen Luftprobe bestimmt. Sollte die Luftqualität des Zielraumes 10 den erwarteten Normen nicht mehr entsprechen, so meldet der Sensor 5 dies der Steuerzentrale 7, welche einen Lüfter 9 zur Luftumwälzung bzw. eine Frischluftzufuhr 11 ansteuert. Wird anschließend eine hinreichend verbesserte Luftqualität gemessen, so schaltet sich der Lüfter 9 bzw. die Frischluftzufuhr 11 wieder ab.

Ferner ist es möglich, in einem Ansaugmelder 8 mehrere unterschiedliche Sensoren zu integrieren, wie beispielsweise ein CO- bzw. CO₂-Sensor 5 in Kombination mit einem Detektor 4 zum Erkennen von Brandkenngrößen oder aber auch ein Sauerstoffmelder 3a in Kombination mit einem der oben genannten anderen Sensoren 4 oder 5.

In der erfindungsgemäßen Vorrichtung ist neben dem Sauerstoffmelder 3a noch ein Referenzsauerstoffmelder 3b zum Messen der Sauerstoffkonzentration der aus dem Zielraum 10 entnommenen Luftprobe eingesetzt wird. Dieser dient als Referenz zum Sauerstoffmelder 3a und sitzt ständig im Luftstrom, aber er ist normalerweise ausgeschaltet. Dadurch wird die Alterung des Referenzsauerstoffmelders 3b vermieden. Der Melder 3b wird in regelmäßigen Abständen (z.B. einmal am Tag oder einmal pro Woche) eingeschaltet. Das Signal zum Einschalten des Referenzsauerstoffmelders 3b wird beispielsweise von einer Zeitschaltuhr erzeugt. Es kann auch auf Tastendruck, etwa bei Wartungen, erzeugt werden. Nach dem Einschalten des Referenzsauerstoffmelders 3b wird eine Mindestheizzeit abgewartet. Danach werden beide Messwerte des Sauerstoffmelders 3a und des Referenzmelders 3b verglichen. Ist der Unterschied zwischen beiden Messwerten größer als eine vorgegebene Schwelle, wird eine Störung gemeldet, und der Referenzsauerstoffmelder 3b wird nicht mehr ausgeschaltet. Seine Messwerte werden anstelle des gealterten Sauerstoffmelders 3a ausgewertet.

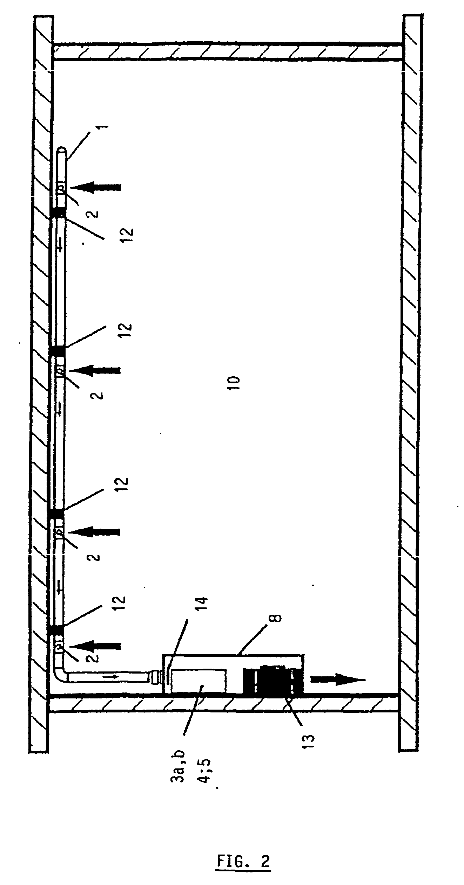

Fig. 2 zeigt eine schematische Darstellung der erfindungsgemäßen Vorrichtung zum Messen des Sauerstoffgehaltes in einem abgeschlossenen Zielraum 10. In der dargestellten Ausführungsform ist das Ansaugrohrsystem 1 unterhalb der Decke des Zielraumes 10 mittels Rohrschellen 12 angebracht. Durch die Ansaugöffnungen 2 in dem Ansaugrohrsystem 1 wird Luft aus dem Zielraum 10 angesaugt. Hierzu wird eine Ansaugeinheit 13, welche in dem Ansaugmelder 8 integriert ist, eingesetzt. Die Ansaugeinheit 13 und das Ansaugrohrsystem 1 werden über einen Luftstromsensor 14, weicher vorzugsweise am Ende des Ansaugrohrsystems 1 plaziert ist, überwacht.

Nachdem die angesaugte Luftprobe den Luftstromsensor 14 passiert hat, umströmt diese den Sauerstoffmelder 3a. Der Sauerstoffmelder 3a misst die Sauerstoffkonzentration der Luftprobe, welche einen Mittelwert der Sauerstoffkonzentration der Luft des Zielraumes 10 repräsentiert. Der Meßwert wird in dem Sauerstoffmelder 3a mit Schwellenwerten verglichen. Beim Überschreiten der Schwellenwerte ist der Sauerstoffgehalt in der Raumluft des Zielraumes 10 zu hoch, um einen Brand sicher zu vermeiden. Beim Auftreten des Signals "Überschreiten eines ersten Schwellenwertes" steuert die Steuerung 7 die Vorrichtung an, die das Inertgas erzeugt und in den Zielraum 10 einleitet (Generator).

Steigt der Sauerstoffgehalt weiter an, ist es ein Zeichen dafür, daß der Generator defekt ist, da er kein Inertgas in den Raum 10 einleitet. Bei Auftreten des Signals "Überschreiten eines zweiten Schwellenwertes" meldet die Steuerung 7 "Störung".

Beim Unterschreiten der Schwellenwerte wird keine Vollinertisierung ausgelöst. Beim Auftreten des Signals "Unterschreiten eines ersten Schwellenwertes" stoppt die Steuerung 7 den Generator, weil der gewünschte Sauerstoffgehalt erreicht ist.

Sinkt der Sauerstoffgehalt weiter, ist es ein Zeichen dafür, daß der Generator defekt ist, da er nicht mehr aufhört, Inertgas in den Zielraum 10 einzuleiten. Beim Auftreten des Signals "Unterschreiten eines zweiten Schwellenwertes meldet die Steuerung 7 "Störung".

Sinkt der Sauerstoffgehalt unter einen für Menschen gefährlichen Wert, sind Maßnahmen zum Personenschutz einzuleiten. Beim Auftreten des Signals "Unterschreiten eines dritten Schwellenwertes" leitet die Steuerung 7 Personenschutzmaßnahmen ein, wie etwa eine Evakuierung des Raumes oder eine Sperre des Zutritts.

Anstelle des Sauerstoffmelders 3a kann in dem Ansaugmelder 8 auch ein CO- bzw. CO₂-Sensor 5 und/oder ein Detektor 4 zum Erkennen von Brandkenngrößen eingesetzt werden.

Es sei darauf hingewiesen, daß die Ausführung der Erfindung nicht auf das in den Figuren 1 und 2 beschriebenen Ausführungsbeispielen beschränkt ist, sondern auch in einer Vielzahl von Varianten möglich ist.

### Bezugszeichenliste

- 1: Ansaugrohrsystem
- 2: Ansaugöffnung
- 3a: Sauerstoffmelder
- 3b: Referenzsauerstoffmelder
- 4: Detektor (zur Erkennung von Brandkenngrößen)
- 5: CO- bzw. CO₂-Sensor
- 6: Anlage (zur Produktion und zum Einleiten von Inertgas)
- 7: Steuerung
- 8: Ansaugmelder
- 9: Lüfter
- 10: Zielraum
- 11: Frischluftzufuhr
- 12: Rohrschelle
- 13: Ansaugeinheit
- 14: Luftstromsensor
- 15: Inertgasvorrichtung zur Brandvermeidung und/oder -löschung

## Patentansprüche

1. Verfahren zum Messen des Sauerstoffgehaltes in einem abgeschlossenen Zielraum (10) zur Überwachung von Inertisierungsniveaus bei einer Inertgasvorrichtung zur Brandvermeidung und/oder -löschung (15), mit folgenden Verfahrensschritten:
a) eine Luftprobe wird über eine Reihe von Ansaugöffnungen (2) eines Ansaugrohrsystems (1) aus dem Zielraum (10) genommen;
b) mit einem Sauerstoffmelder (3a, 3b) wird die Sauerstoffkonzentration der angesaugten. Luftprobe bestimmt.
**gekennzeichnet durch**
folgende Verfahrensschritte nach Verfahrensschritt b):
b1) die Sauerstoffkonzentration in der angesaugten Luftprobe wird mit einem Referenzsauerstoffmelder (3b) bestimmt;
b2) der Messwert, der in Verfahrensschritt b) bestimmten Sauerstoffkonzentration der angesaugten Luftprobe wird mit dem Messwert der Sauerstoffkonzentration des Referenzsauerstoffmelders (3b) verglichen; und
b3) beim Überschreiten der Abweichung des Messwertes der Sauerstoffkonzentration des Sauerstoffmelders (3a) von dem Messwert der Sauerstoffkonzentration des Referenzsauserstoffmelders (3b) gibt der Sauerstoffmelder (3a) oder der Referenzsauerstoffmelder (3b) ein Störungssignal ab,
wobei im Verfahrensschritt b1) zur Verhinderung der Alterung des Referenzsauerstoffmelders (3b) der Referenzsauerstoffmelder (3b) in regelmäßigen Zeitabständen nur kurzzeitig eingeschaltet wird.

2. **Verfahren nach Anspruch 1,**
**gekennzeichnet durch**
folgende zusätzliche Verfahrensschritte nach Verfahrensschritt b):
c) der Messwert der Sauerstoffkonzentration der angesaugten Luftprobe wird
im Sauerstoffmelder (3a, 3b) mit eingestellten Schwellenwerten verglichen; d) beim Überschreiten des eingestellten Schwellenwertes erfolgt **durch** Einleiten von Inertgas in den Zielraum (10) eine Absenkung der Sauerstoffkonzentration.

3. Verfahren nach Anspruch 1 oder 2,
**gekennzeichnet durch**
folgende weitere Verfahrensschritte vor oder mit Verfahrensschritt b):
b4) in der angesaugten Luftprobe werden mit einem Detektor (4) Brandkenngrößen gemessen;
b5) der Detektor (4) gibt beim Erkennen einer Brandkenngröße ein Signal für eine Vollinertisierung des Zielraumes (10) ab.

4. Verfahren nach Anspruch 3,
**dadurch gekennzeichnet, dass**
die im Detektor (4) nachgewiesenen Brandkenngrößen Rauch in Form von Partikeln, Aerosolen oder Dampf und wenigstens ein Brandgas sind.

5. Verfahren nach Anspruch 4,
**dadurch gekennzeichnet, dass**
das im Detetctor (4) nachgewiesene Brandgas CO oder CO₂ ist.

6. Verfahren nach einem der vorhergehenden Ansprüche,
**gekennzeichnet durch**
folgende weitere Verfahrensschritte nach Verfahrensschritt a):
b6) in der angesaugten Luftprobe wird mit einem CO- und/oder CO₂-Sensor (5) der CO- und/oder CO₂-Gehalt gemessen;
b7) in Abhängigkeit des Messwertes des CO- und/oder CO₂-Gehaltes wird dem Zielraum (10) Frischluft zugeführt.

7. Verfahren nach einem der vorhergehenden Ansprüche,
**gekennzeichnet durch**
folgenden Verfahrensschritt nach Verfahrensschritt b3):
b8) der Referenzsauerstoffmelder (3b) bestimmt nach Abgabe des Störungssignals kontinuierlich die Sauerstoffkonzentration in der angesaugten Luftprobe, wobei die weitere Auswertung des Messwertes der Sauerstoffkonzentration in Verfahrensschritt c) anhand des **durch** den Referenzsauerstoffmelders (3b) bestimmten Messwertes anstelle des **durch** den Sauerstoffmelder (3a) bestimmten Messwertes erfolgt.

8. Vorrichtung zur Durchführung des Verfahrens nach einem der Ansprüche 1 bis 7, mit:
- einer Inertgasvorrichtung zur Brandvermeidung und/oder -löschung (15) in einem geschlossenen Raum (10);
- wenigstens einem Ansaugrohrsystem (1) zum Ansaugen einer Luftprobe über verschiedene Ansaugöffnungen (2) aus dem zu überwachenden Zielraum (10);
- wenigstens einem Sauerstoffmelder (3a) zum Messen der Sauerstoffkonzentration in einer aus dem Zielraum (10) entnommenen Luftprobe;
- einer Steuerung (5), über welche die Einstellung von Inertisierungsniveaus im Zielraum (10) und die Steuerung der Frischluftzufuhr (11) bzw. des Lüfters (9) erfolgt;
**gekennzeichnet durch**
einen Referenzsauerstoffmelder (3b) zum Messen der Sauerstoffkonzentration in der aus dem Zielraum (10) entnommenen Luftprobe als Referenz zum Sauerstoffmelder (3a), wobei beim Überschreiten der Abweichung des Messwertes der Sauerstoffkonzentration des Sauerstoffmelders (3a) von dem Messwert der Sauerstoffkonzentration des Referenzsauserstoffmelders (3b) der Sauerstoffmelder (3a) oder der Referenzsauerstoffmelder (3b) ein Störungssignal abgibt, und wobei der Referenzsauerstoffmelder (3b) in regelmäßigen Zeitabständen nur kurzzeitig eingeschaltet wird, um die Alterung des Referenzsauerstoffmelders (3b) zu verhindern,

9. Vorrichtung nach Anspruch 8,
**gekennzeichnet durch**
wenigstens einen Detektor (4) zum Erkennen von Brandkenngrößen in einer von einem der Ansaugrohrsysteme (1) aus dem Zielraum (10) genommenen Luftprobe;

10. Vorrichtung nach Anspruch 8 oder 9,
**gekennzeichnet durch**
wenigstens einen CO- bzw. CO₂-Sensor (5) zum Messen der Luftqualität in einer von einem der Ansaugrohrsysteme (1) aus dem Zielraum (10) genommenen Luftprobe.

11. Vorrichtung nach einem der Ansprüche 8 bis 10,
**dadurch gekennzeichnet, dass**
wenigstens einer der Sauerstoffmelder (3a, 3b) und/oder wenigstens einer der Detektoren (4) und/oder wenigstens einer der CO- bzw. CO₂-Sensoren (5) zusammen in einem der Ansaugrohrsysteme (1) integriert sind.

12. Vorrichtung nach einem der Ansprüche 8 bis 11,
**dadurch gekennzeichnet, dass** s
als Sauerstoffmelder (3a, 3b) elektrochemische Zellen aus Zirkondioxid eingesetzt werden.

## Revendications

1. Procédé de mesure de la teneur en oxygène dans un espace cible fermé (10) pour surveiller des niveaux d'inertisation dans un dispositif à gaz inerte (15) destiné à éviter et/ou à éteindre un incendie, comprenant les étapes suivantes :
a) on prélève dans l'espace cible (10) un échantillon d'air via une série d'ouvertures d'aspiration (2) d'un système de tubes d'aspiration (1) ;
b) on détermine la concentration d'oxygène de l'échantillon d'air aspiré au moyen d'un détecteur d'oxygène (3a, 3b),
**caractérisé par**
les étapes suivantes à la suite de l'étape b) :
b1) on détermine la concentration d'oxygène dans l'échantillon d'air aspiré au moyen d'un détecteur d'oxygène de référence (3b),
b2) on compare la valeur de mesure de la concentration d'oxygène de l'échantillon d'air aspiré, déterminée dans l'étape b), à la valeur de mesure de la concentration d'oxygène du détecteur d'oxygène de référence (3b) ; et
b3) en cas de dépassement d'un écart entre la valeur de mesure de la concentration d'oxygène du détecteur d'oxygène (3a) et la valeur de mesure de la concentration d'oxygène du détecteur d'oxygène de référence (3b), le détecteur d'oxygène (3a) ou le détecteur d'oxygène de référence (3b) émet un signal de perturbation,
et dans l'étape b1), en vue d'empêcher le vieillissement du détecteur d'oxygène de référence (3b), on met en marche le détecteur d'oxygène de référence (3b) seulement brièvement à des intervalles temporels réguliers.

2. Procédé selon la revendication 1,
**caractérisé par**
les étapes supplémentaires suivantes à la suite de l'étape b) :
c) dans le détecteur d'oxygène (3a, 3b), on compare la valeur de mesure de la concentration d'oxygène de l'échantillon d'air aspiré à des valeurs seuil réglées ;
d) en cas dépassement de la valeur seuil réglée, il se produit un abaissement de la concentration d'oxygène par introduction de gaz inerte dans l'espace cible (10).

3. Procédé selon la revendication 1 ou 2,
**caractérisé par**
les autres étapes suivantes avant ou pendant l'étape b) :
b4) on mesure des grandeurs caractéristiques d'incendie dans l'échantillon d'air aspiré au moyen d'un détecteur (4) ;
b5) en cas de reconnaissance d'une grandeur caractéristique d'incendie, le détecteur (4) émet un signal pour une inertisation totale de l'espace cible (10).

4. Procédé selon la revendication 3,
**caractérisé en ce que**
les grandeurs caractéristiques d'incendie mises en évidence dans le détecteur (4) sont des fumées sous forme de particules, des aérosols ou de la vapeur et au moins un gaz d'incendie.

5. Procédé selon la revendication 4,
**caractérisé en ce que**
le gaz d'incendie mis en évidence dans le détecteur (4) est du CO ou du CO₂.

6. Procédé selon l'une des revendications précédentes,
**caractérisé par**
les autres étapes suivantes, après l'étape a) :
b6) on mesure la teneur en CO et/ou en CO₂ dans l'échantillon d'air aspiré au moyen d'un détecteur de CO et/ou de CO₂ (5) ;
b7) en fonction de la valeur de mesure de la teneur en CO et/ou en CO₂, on amène de l'air frais à l'espace cible (10).

7. Procédé selon l'une des revendications précédentes,
**caractérisé par**
l'étape suivante, après l'étape b3) :
b8) après avoir émis le signal de perturbation, le détecteur d'oxygène de référence (3b) détermine en continu la concentration d'oxygène dans l'échantillon d'air aspiré, et la poursuite de l'évaluation de la valeur de mesure de la concentration d'oxygène dans l'étape c) s'effectue en se basant sur la valeur de mesure déterminée par le détecteur d'oxygène de référence (3b), à la place de la valeur de mesure déterminée par le détecteur d'oxygène (3a).

8. Appareil pour mettre en oeuvre le procédé selon l'une des revendications 1 à 7, comportant
- un dispositif à gaz inerte (15) destiné à éviter et/ou à éteindre un incendie dans un espace fermé (10) ;
- au moins un système de tubes d'aspiration (1) pour aspirer un échantillon d'air via différentes ouvertures d'aspiration (2) hors de l'espace cible (10) à surveiller ;
- au moins un détecteur d'oxygène (3a) pour mesurer la concentration d'oxygène dans un échantillon d'air prélevé dans l'espace cible (10) ;
- une commande (5) via laquelle s'effectue le réglage de niveaux d'inertisation dans l'espace cible (10) et la commande de l'amenée d'air frais (11) ou du ventilateur (9) ;
**caractérisé par**
un détecteur d'oxygène de référence (3b) pour mesurer la concentration d'oxygène dans l'échantillon d'air prélevé dans l'espace cible (10) à titre de référence par rapport au détecteur d'oxygène (3a), et en cas de dépassement d'un écart entre la valeur de mesure de la concentration d'oxygène du détecteur d'oxygène (3a) et la valeur de mesure de la concentration d'oxygène du détecteur d'oxygène de référence (3b), le détecteur d'oxygène (3a) ou le détecteur d'oxygène de référence (3b) émet un signal de perturbation, le détecteur d'oxygène de référence (3b) étant mis en marche uniquement brièvement à des intervalles temporels réguliers, afin d'empêcher le vieillissement du détecteur d'oxygène de référence (3b).

9. Dispositif selon la revendication 8,
**caractérisé par**
au moins un détecteur (4) pour reconnaître des grandeurs caractéristiques d'incendie dans un échantillon d'air prélevé dans l'un des systèmes de tubes d'aspiration (1) provenant de l'espace cible (10).

10. Dispositif selon la revendication 8 ou 9,
**caractérisé par**
au moins un détecteur de CO ou de CO₂ (5) pour mesurer la qualité de l'air dans un échantillon d'air prélevé dans l'un des systèmes de tubes d'aspiration (1) provenant de l'espace cible (10).

11. Dispositif selon l'une des revendications 8 à 10,
**caractérisé en ce que**
l'un au moins des détecteurs d'oxygène (3a, 3b) et/ou l'un au moins des détecteurs (4) et/ou l'au moins des détecteurs de CO ou CO₂ (5) sont intégrés conjointement dans l'un des systèmes de tubes d'aspiration (1).

12. Dispositif selon l'une des revendications 8 à 11,
**caractérisé en ce que**
il est prévu à titre de détecteurs d'oxygène (3a, 3b) des cellules électrochimiques en dioxyde de zirconium.

## Claims

1. Method of measuring the oxygen content of a sealed target space (10) in order to monitor inertisation levels in an inert gas device (15) for preventing and/or extinguishing fires, comprising the following steps:
a) an air sample is taken from the target space (10) via a series of suction openings (2) of a suction pipe system (1), and
b) the oxygen concentration of the air sample is determined by means of an oxygen sensor (3a, 3b),
**characterised by** the following steps after step b):
b1) the oxygen concentration of the air sample is determined by means of a reference oxygen sensor (3b);
b2) the measured value for the oxygen concentration of the air sample determined in step b) is compared with the measured value for the oxygen concentration of the reference oxygen sensor (3b), and
b3) if the deviation of the measured value for the oxygen concentration of the oxygen sensor (3a) from the measured value for the oxygen concentration of the reference oxygen sensor (3b) exceeds a preset value, the oxygen sensor (3a) or the reference oxygen sensor (3b) emits an alarm signal,
the reference oxygen sensor (3b) being switched on only for short periods at regular intervals in step b1) in order to prevent ageing of the reference oxygen sensor (3b).

2. Method according to claim 1, **characterised by** the following additional steps after step b):
c) the measured value for the oxygen concentration of the air sample is compared with preset threshold values in the oxygen sensor (3a, 3b), and
d) if the preset threshold value is exceeded, the oxygen concentration is reduced by introducing inert gas into the target space (10).

3. Method according to claim 1 or claim 2, **characterised by** the following further steps before or with step b):
b4) fire parameters in the air sample are measured by means of a detector (4);
b5) the detector (4) emits a signal for complete inertisation of the target space (10) when a fire parameter is detected.

4. Method according to claim 3, **characterised in that** the fire parameters detected in the detector (4) are smoke in the form of particles, aerosols or vapour and at least one combustion gas.

5. Method according to claim 4, **characterised in that** the combustion gas detected in the detector (4) is CO or CO₂.

6. Method according to one of the preceding claims, **characterised by** the following further steps after step a):
b6) the CO and/or CO₂ content of the air sample is measured by means of a CO and/or CO₂ sensor (5), and
b7) fresh air is supplied to the target space (10) as a function of the measured value for the CO and/or CO₂ content.

7. Method according to one of the preceding claims, **characterised by** the following step after step b3):
b8) the reference oxygen sensor (3b) continuously determines the oxygen concentration of the air sample following the output of the alarm signal, further evaluation of the measured value for the oxygen concentration in step c) being effected by way of the measured value determined by the reference oxygen sensor (3b) instead of the measured value determined by the oxygen sensor (3a).

8. Device for carrying out the method according to one of claims 1 to 7, comprising:
- an inert gas device (15) for preventing and/or extinguishing fires in a closed space (10);
- at least one suction pipe system (1) for taking an air sample from the target space (10) to be monitored via various suction openings (2);
- at least one oxygen sensor (3a) for measuring the oxygen concentration of an air sample taken from the target space (10), and
- a control unit (5) for setting inertisation levels in the target space (10) and controlling the fresh air supply (11) or the fan (9),
**characterised by** a reference oxygen sensor (3b) for measuring the oxygen concentration of the air sample taken from the target space (10) as a reference relative to the oxygen sensor (3a), wherein, if the deviation of the measured value for the oxygen concentration of the oxygen sensor (3a) from the measured value for the oxygen concentration of the reference oxygen sensor (3b) exceeds a preset value, the oxygen sensor (3a) or the reference oxygen sensor (3b) emits an alarm signal and wherein the reference oxygen sensor (3b) is switched on only for short periods at regular intervals in order to prevent ageing of the reference oxygen sensor (3b).

9. Device according to claim 8, **characterised by** at least one detector (4) for detecting fire parameters in an air sample taken from the target space (10) by one of the suction pipe systems (1).

10. Device according to claim 8 or claim 9, **characterised by** at least one CO or CO₂ sensor (5) for measuring the air quality of an air sample taken from the target space (10) by one of the suction pipe systems (1).

11. Device according to one of claims 8 to 10, **characterised in that** at least one of the oxygen sensors (3a, 3b) and/or at least one of the detectors (4) and/or at least one of the CO or CO₂ sensors (5) is/are integrated into one of the suction pipe systems (1).

12. Device according to one of claims 8 to 11, **characterised in that** electrochemical cells made of zirconium dioxide are used as the oxygen sensors (3a, 3b).
